(19) Europäisches Patentamt
European Patent Office
Office européen des brevets

(11) Veröffentlichungsnummer: **0 283 753**
**B1**

(12) # EUROPÄISCHE PATENTSCHRIFT

(45) Veröffentlichungstag der Patentschrift: **19.09.90**

(51) Int. Cl.[5]: **C07D 301/12**
// B01J31/34

(21) Anmeldenummer: **88102769.2**

(22) Anmeldetag: **25.02.88**

(54) **Verfahren zur katalytischen Epoxidation von Olefinen mit Wasserstoffperoxid.**

(30) Priorität: **25.03.87 DE 3709829**

(43) Veröffentlichungstag der Anmeldung:
**28.09.88 Patentblatt 88/39**

(45) Bekanntmachung des Hinweises auf die Patenterteilung:
**19.09.90 Patentblatt 90/38**

(84) Benannte Vertragsstaaten:
**AT BE CH DE FR GB IT LI NL SE**

(56) Entgegenhaltungen:
**EP-A- 0 109 273**
**EP-A- 0 232 752**
**DE-A- 3 539 268**

**CHEMICAL ABSTRACTS, Band 103, Nr. 7, 19.08.1985, Columbus, Ohio, USA BORTOLINI, O.; DI FURIA, F.; MODENA, G.; SERAGLIA, R. "Metal catalysis in oxidation by peroxides. Sulfide oxidation and olefin epoxidation by dilute hydrogen peroxide, catalyzed by molebdenum and tungsten derivatives under phase-transfer conditions", Seite 515, Spalte 1, Zusammenfassung-Nr. 53 336r.**
**F. VARESCON, THESE, L'UNIVERSITE CLAUDE BERNARD LYON I, 1982.**

(73) Patentinhaber: **Degussa Aktiengesellschaft, Weissfrauenstrasse 9, D-6000 Frankfurt am Main 1(DE)**

(72) Erfinder: **Schmidt, Manfred, Dr., Unter-Haitzergasse 19, D-6460 Gelnhausen(DE)**
Erfinder: **Prescher, Günter, Dr., Liesingstrasse 2, D-6450 Hanau 9(DE)**
Erfinder: **Buchler, Johann, Prof. Dr., Richard-Wagner-Weg 93, D-6100 Darmstadt(DE)**
Erfinder: **Kleemann, Axel, Prof. Dr., Bornweg 36, D-6052 Mühlheim 2(DE)**

**EP 0 283 753 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur katalytischen Epoxidation von Olefinen mit Wasserstoffperoxid in Anwesenheit eines Übergangsmetallporphyrinkomplexes, bei dem ein erforderlicher Ladungsausgleich durch ein Anion erfolgt.

Olefinoxide (Oxirane) sind Verbindungen von beträchtlicher industrieller Bedeutung. Sie finden Verwendung auf dem Gebiet der Lacke, zur Herstellung von Polyethern, Polyurethanen, Epoxidharzen, Detergentien, Glykolen und einer Vielzahl von organischen Zwischenprodukten (siehe US-PS 2 412 136 sowie DE-AS 11 39 477).

Zur Epoxidation von Olefinen sind schon verschiedene Verfahren bekannt. So lassen sich Oxirane nach der Chlorhydrinmethode durch Umsetzung von Olefinen mit Chlor oder Natriumhypochlorit in alkalischem Medium und nachfolgender Behandlung mit Basen herstellen. Ein grundsätzlicher Nachteil dieses Verfahrens besteht in der Bildung salzhaltiger umweltbelastender Abwässer und unerwünschter chlorierter Nebenprodukte (siehe Ullmann's Enzyklopädie der technischen Chemie, Band 10, Seite 565 (3. Auflage)).

Ein weiterer Prozeß beruht auf der Umsetzung von Olefinen mit organischen Hydroperoxiden in Gegenwart eines Katalysators (siehe DE-AS 14 68 012). Dieses zweite Syntheseprinzip hat den entscheidenden Nachteil, daß aufgrund der Stöchiometrie der Epoxidationsreaktion das üblicherweise teure organische Hydroperoxid (ROOH, wobei R z. B. einen niedermolekularen Rest, wie t-Butyl oder Cumyl, bedeuten kann) während der Reaktion gemäß

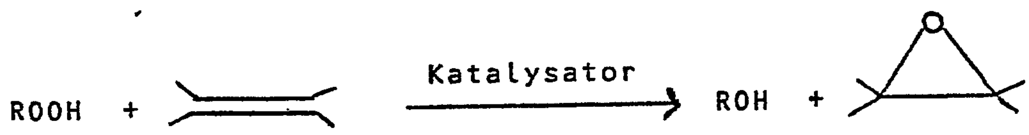


in große Mengen des entsprechenden Alkohols (ROH) umgewandelt wird. Falls der entsprechende Alkohol nicht verwertet werden kann, muß er vom Verfahrensprodukt abgetrennt und entsorgt oder über mehrere Verfahrensstufen in das entsprechende Hydroperoxid zurückverwandelt werden, wodurch das Epoxidationsverfahren auch in wirtschaftlicher Hinsicht aufwendig wird.

Ein weiteres Verfahren beruht auf der Verwendung von organischen Persäuren, die man durch Luftoxidation der entsprechenden Aldehyde oder aus Carbonsäuren mit Wasserstoffperoxid erhält (siehe BE-PS 535 068). Der Einsatz dieser organischen Percarbonsäuren ist wegen deren Zersetzlichkeit stets mit einem Risiko behaftet und erfordert deshalb aufwendige Vorsichtsmaßnahmen in bezug auf Verfahrensführung und Apparateaufbau. Zusätzlich entstehen bei Epoxidierungen mit organischen Persäuren immer große Mengen der entsprechenden Carbonsäuren, die in stöchiometrischer Menge oder überstöchiometrischer Menge abgetrennt und entsorgt oder zurückgeführt werden müssen.

Die geschilderten Nachteile lassen sich durch Verwendung von Wasserstoffperoxid als Epoxidationsmittel beheben, da hierbei nach der Theorie neben dem Epoxidationsprodukt nur Wasser anfallen sollte. Da die Reaktivität des Wasserstoffperoxids gegenüber Olefinen schwach ist, werden Epoxidationen mit diesem Reagens unter Verwendung von Katalysatoren durchgeführt. Nur für wenige Olefine sind Katalysatoren wie Molybdän- und Wolframverbindungen geeignet. In diesem Zusammenhang wird beispielsweise hingewiesen auf GB-PS 837 464, bei welchem die in J.A.C.S., Band 59, Seiten 2342 bis 2344 (1937) beschriebenen verschiedenen Metallkatalysatoren verwendet werden, auf US-PS 2 786 854, wonach Wolframsäure eingesetzt wird, auf US-PS 2 833 787, wonach saure Salze von Metallen aus der Gruppe VI des Periodensystems der Elemente, z. B. von Wolfram oder Molybdän, angewandt werden, auf BE-PS 860 776, wonach Wolfram- und Molybdän-haltige Verbindungen verwendet werden, auf US-PS 3 993 673, wonach Arsen-haltige Katalysatoren verwendet werden, auf US-PS 3 953 362, wonach ein Molybdän-haltiger Katalysator angewandt wird, auf US-PS 4 026 908, wonach Quecksilberderivate plus eine Molybdän-, Wolfram-, Vanadin- oder Titanverbindung angewandt wird, auf US-PS 3 806 467, wonach organische und anorganische Zinnverbindungen plus organische oder anorganische Verbindungen, die Molybdän, Wolfram, Vanadin, Selen oder Bor enthalten, eingesetzt werden, auf Bull. Chem. Soc. Jap. 42, Seiten 1604 (1969), wonach Selendioxid angewandt wird und auf US-PS 3 778 451, wonach Molybdän-, Wolfram-, Vanadin-, Niob-, Tantal-, Uran- und Rheniumverbindungen eingesetzt werden.

Diese Stoffe sind zwar katalytisch aktiv, doch haben aus verschiedenen Gründen die damit grundsätzlich ausführbaren Verfahren keinen Eingang in die Technik gefunden. In Verbindung mit Wasserstoffperoxidlösungen wird durch sie entweder das Wasserstoffperoxid rasch zersetzt oder nur eine unbefriedigende Epoxidationsgeschwindigkeit erreicht. Verfahren mit diesen Katalysatoren sind auch insoweit problematisch, als neben dem gewünschten Epoxidationsprodukt häufig größere Mengen an Nebenprodukten, wie Diole und Ketone gebildet werden, deren Abtrennung erhebliche Schwierigkeiten bereiten kann.

Es sind auch schon Versuche unternommen worden, Verfahren zur katalytischen Epoxidation von Olefinen mit anderen Epoxidationsmitteln unter Verwendung von Metallporphyrinkomplexen als Katalysatoren durchzuführen. Als Epoxidationsmittel wurden hierbei Verbindungen wie Jodosobenzol (PhJO) (Groves, J.T.; Nemo, T.E.; Myers, R.S., J. Am. Chem. Soc., 101, 1032 (1979), Alkalimetallhypochlorit, wie NaOCl oder LiOCl (Guilmet, E.; Meunier, B.; Tetrahedron Lett. 1980, 4449) sowie organische Hydroperoxide, wie t-Butylhydroperoxid oder Cumolhydroperoxid (Ledon, H.J.; Durbut, P.; Varescon, F., J. Am. Chem. Soc. 103, 3601 (1981) eingesetzt. Als zur Umsetzung mit diesen Epoxidationsmitteln geeignete Metallkatalysatoren sind z. B. das Chloro-Eisen(III)-tetraphenylporphyrin (ClFe(TPP)), das Chloro-Mangan (III)-tetraphenylporphyrin (ClMn(TPP)) oder das Chloro-Chrom(III)-tetraphenylporphyrin (ClCr(TPP)) vorgeschlagen worden. Mangan(III)-tetraphenylporphyrin wurde auch bereits mit Wasserstoffperoxid als Oxidationsmittel eingesetzt (Renaud, J.-P.; Battioni, P.; Bartoli,J.F.; Mansuy, D., J. Chem. Soc., Chem. Commun., 1985, 888). Allerdings wirken diese Katalysatoren stark zersetzend auf $H_2O_2$, so daß die bezüglich Wasserstoffperoxid erreichbaren Selektivitäten nur sehr gering sind, es sei denn, es werden aufwendig substituierte Porphyrinliganden verwendet.

Auch Oxo-Metallporphyrinkomplexe, wie Oxo-chloro(5,10,15, 20-tetraphenylporphyrin)-molybdän(V) (O=Mo(TPP)Cl) sind in Verbindung mit organischen Hydroperoxiden schon vorgeschlagen worden. Ein Versuch, anstelle eines organischen Hydroperoxids Wasserstoffperoxid mit einem Katalysator der Zusammensetzung Oxo(5,10,15,20-tetraphenylporphyrin)-molybdän(V)-methoxid zur Epoxidierung des Olefins Cyclohexen zu verwenden, schlug jedoch fehl: Es konnte keine Epoxidation beobachtet werden (F.Varescon, These, L'Université Claude Bernard-Lyon I, 1982).

Es wurde nun gefunden, daß die katalytische Epoxidation von Olefinen mit Wasserstoffperoxid mit sehr hoher Selektivität gelingt, wenn das Olefin in Gegenwart von

- Wolfram-oxo-Komplexen oder
- einer zweikernigen Verbindung des Typs µ-Oxobis [porphyrinatooxowolfram(V)] oder
- einer Oxoperoxoverbindung des Typs cis-Oxoperoxowolfram(VI)porphyrin oder
- eines cis-Dioxoporphyrinatowolfram(VI)-Komplexes

mit jeweils

- Oktaethylporphyrin oder
- 5,10,15,20-Tetraphenylporphyrin oder
- 5,10,15,20-Tetra(4-pyridyl)-porphyrin

als Liganden, bei denen gegebenenfalls jeweils Wasserstoffatome oder freie Elektronenpaare an den Phenyl- bzw. Pyridylgruppen ein- oder mehrmals substituiert sind durch Halogen, Hydroxy, Carboxy, Cyano, Rhodano, Nitro, $C_1$-$C_6$-Alkyl, Trihalogenmethyl, $C_1$-$C_6$-Alkoxy,$C_1$-$C_6$-Alkansulfonyloxy, Aminocarbonyl, einen oder zwei $C_1$-$C_6$-Alkylreste enthaltendes Aminocarbonyl, $C_1$-$C_6$-Alkylcarbonyl, Amino, Di-$C_1$-$C_6$-Alkylamino, $C_1$-$C_6$-Alkanoylamino, $C_1$-$C_6$-Alkyl-$C_1$-$C_6$-alkanoylamino, $C_1$-$C_6$-Alkansulfonylamino, $C_1$-$C_6$-Alkyl-$C_1$-$C_6$-alkansulfonylamino, Aminosulfonyl, einen oder zwei $C_1$-$C_6$-Alkylreste enthaltendes Aminosulfonyl, $C_1$-$C_6$-Alkoxysulfonyl (-$SO_2$-O-$C_1$-$C_6$-Alkyl), Sulfo oder $C_1$-$C_6$-Alkansulfonyl und zwei dieser Reste auch die Methylendioxygruppe sein können, wobei der Komplex bei dem Wolfram-oxo-Komplexen gegebenenfalls am Zentralatom ein Anion aus der Reihe $F^-$, $Cl^-$, $Br^-$, $J^-$, $CH_3O^-$, $C_2H_5O^-$, $C_3H_7O^-$, t-$C_4H_9O^-$, $HO^-$, $AcO^-$, $SCN^-$, $CN^-$, $C_6H_5O^-$ trägt, in homogener Phase oder in einem Zweiphasensystem mit Wasserstoffperoxid umgesetzt wird.

Durch die sterischen und elektronischen Effekte der genannten Substituenten am Phenyl- bzw. Pyridylrest des 5,10,15,20-Tetraphenylporphyrins bzw. 5,10,15 20- Tetra(4-pyridyl)porphyrins kann man, angepaßt an das jeweilige Olefin, die katalytischen Eigenschaften steuern und optimieren.

Die erfindungsgemäß zur Durchführung des Verfahrens vorgesehenen Katalysatoren sind zum Teil neue Stoffe. Darunter fallen alle für das erfindungsgemäße Verfahren beanspruchten Wolfram-oxo-Komplexe mit Ausnahme derjenigen, welche $OH^-$, $CH_3O^-$ oder $C_6H_5O^-$ als Anion tragen. Die schon bekannten Komplexe sind nach den bekannten Literaturmethoden in großer Reinheit zugänglich (J.W. Buchler et al., Chem. Ber., 1973, 106, 2710; Liebigs Ann. Chem., 1971, 745, 135; Inorg. Nucl. Chem. Lett., 1972, 8, 1073, K. Rohbock, Dissertation, RTWH Aachen, 1972).

Die verschiedenen Porphyrinliganden werden, sofern sie nicht käuflich sind, nach Adler et al., J. Org. Chem. 32, 476 (1967) bzw. Adler et al., J. Heterocycl. Chem. 5, 669 (1968) dargestellt und sofern erforderlich, von Chlorin (Porphyrin mit 1 teilhydrierten Pyrrolglied) befreit (K.M. Smith et al., Tetrahedron Lett. 30, 2887, (1973)).

Die neuen, in der gleichzeitig eingereichten EP-A 0 283 754 beschriebenen Stoffe unter den Wolfram-oxo-Komplexen können nur zum Teil nach der bekannten $W(CO)_6$-Methode hergestellt werden.

In der genannten Parallelanmeldung EP-A 0 283 754 sind neue Herstellungsverfahren für diese neuen Stoffe bzw. ihre Vorstufe erstmals offenbart, welche sich auch zur Gewinnung der bereits bekannten, für das erfindungsgemäße Verfahren beanspruchten Komplexe eignen.

Das erste Verfahren ist dadurch gekennzeichnet, daß man ein Mol 5,10,15,20-Tetraphenylporphyrin oder 5,10,15,20-Tetra(4-pyridyl)-porphyrin mit mindestens einem Mol Wolframpentahalogenid und 1 Mol Wasser in einem hochsiedenden, alle Reaktanden lösenden Solvens beim Temperaturen von 160–250 °, vorzugsweise 180–220°C, umsetzt, das Solvens vorzugsweise im Vakuum entfernt, den Rückstand nach Aufnehmen in einem organischen Lösungsmittel durch Säulenchromatographie fraktioniert, dann die in der Hauptfraktion vorliegenden, hinsichtlich der Zusammensetzung des Anions noch inhomogenen Komplexe entweder jeweils mit einer äquiolaren Menge einer gegebenenfalls in situ erzeugten Alkalime-

zugsweise 40–60°C, in einem Lösungsmittelgemisch, das sowohl den Wolfram-oxo-Komplex als auch die Alkalimetallverbindung löst, umsetzt und daraus die das gewünschte Anion tragenden Wolfram-oxo-Komplexe durch Einengen zur Kristallisation bringt oder aber jeweils mit einem Überschuß der zum gewünschten Anion korrespondierenden Brönsted-Säure bei 20–180, vorzugsweise 40–120°C, in Lösungsmittelgemischen behandelt, die sowohl den Wolfram-oxo-halogeno-Komplex als auch die Brönstedsäure lösen, und daraus die das gewünschte Anion tragenden Wolfram-oxo-Komplexe durch Einengen zur Kristallisation bringt.

Ein weiterer neuer Herstellungsweg für Wolfram-oxo-Komplexe allgemein, insbesondere aber für solche gemäß der Erfindung besteht darin, daß man in einer ersten Stufe 2 Mol Wolframhexahalogenid mit 1 Mol Wolframhexacarbonyl in einem Überschuß eines koordinierenden Lösungsmittels L zur Vorstufe $WX_4L_2$ umsetzt und dann in zweiter Stufe mindestens 4 Mol der nicht isolierten Vorstufe mit 4 Mol des jeweiligen Porphyrinliganden, sowie 1 Mol Sauerstoff und 2 Mol Wasser in dem genannten Lösungsmittel bei Temperaturen von 160–250°, vorzugsweise 180–220°C umsetzt, das Solvens vorzugsweise im Vakuum entfernt, den Rückstand nach Aufnehmen in einem organischen Lösungsmittel durch Säulenchromatographie fraktioniert, die in der Hauptfraktion vorliegenden, hinsichtlich der Zusammensetzung des Anions noch inhomogenen Komplexe entweder jeweils mit einer äquimolaren Menge einer gegebenenfalls in situ erzeugten Alkalimetallverbindung, welche das gewünschte Anion in Bindung mit dem Alkalimetall enthält, bei 0–100°, vorzugsweise 40–60°C, in einem Lösungsmittelgemisch, das sowohl den Wolfram-oxo-Komplex als auch die Alkalimetallverbindung löst, umsetzt und daraus die das gewünschte Anion tragenden Wolfram-oxo-Komplexe durch Einengen zur Kristallisation bringt oder aber jeweils mit einem Überschuß der zum gewünschten Anion korrespondierenden Brönsted-Säure bei 20–180°, vorzugsweise 40–120°C, in Lösungsmittelgemischen behandelt, die sowohl den Wolfram-oxo-halogeno-Komplex als auch die Brönstedsäure lösen, und daraus die das gewünschte Anion tragenden Wolfram-oxo-Komplexe durch Einengen zur Kristallisation bringt.

Den ersten Syntheseweg zeigt die Gleichung (1), den zweiten Syntheseweg die Gleichung (2a) und (2b) und die Überführung der in dem zweiten Syntheseweg zugänglichen Komplexe mit Halogen als Anion in die übrigen Komplexverbindungen die Gleichung (3)

$$(1)\quad H_2(P) + WX_5 + H_2O \longrightarrow WO(P)X + 4HX;$$

$$(2a)\quad 2WX_6 + W(CO)_6 + 6PhCN \longrightarrow 3WX_4(PhCN)_2 + 6CO;$$

$$(2b)\quad 4H_2(P) + 4WX_4(PhCN)_2 + O_2 + 2H_2O \longrightarrow 4WO(P)X + 12HX + 8PhCN;$$

$$(3)\quad WO(P)X + MX' \longrightarrow WO(P)X' + MX.$$

Bedeutungen:
P = Porphyrinligand, gegebenenfalls substituiert;
X, X′ = beliebiges, einfach negativ geladenes Anion;
M = Alkalimetall oder Proton.

Nach dem erfindungsgemäßen Epoxidationsverfahren können Olefine entsprechend der allgemeinen Formel

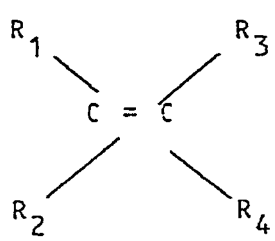

umgesetzt werden, wobei $R_1$ bis $R_4$ identisch oder verschieden sein können und sowohl Wasserstoff oder einen linearen oder verzweigten Alkylrest mit 1 bis 30 Kohlenstoffatomen oder einen Cycloalkylrest mit 3 bis 12 C-Atomen, die als Heteroatome z.B. ein- oder mehrere O-, N- oder S-Atome enthalten können, bedeuten. $R_1$ und $R_2$ oder $R_3$ und $R_4$ können auch durch funktionelle Gruppen substituiert sein, welche im Reaktionsmilieu stabil sind, wie z.B. durch Hydroxy-, Chloro-, Fluoro-, Bromo-, Jodo-, Nitro-,

che im Reaktionsmilieu stabil sind, wie z.B. durch Hydroxy-, Chloro-, Fluoro-, Bromo-, Jodo-, Nitro-, Methoxy-, Alkoxy-, Amino-, Carbonyl-, Ester-, Amido-, Nitrilo-Gruppen. Sie können auch ungesättigt sein, d.h. daß Polyolefine, wie z.B. Diene, Triene und andere Verbindungen mit Doppelbindungen ebenfalls in der vorliegenden Erfindung verwendet werden können, seien sie konjugiert oder nicht. Unter dieser Voraussetzung kommen unter der Olefinen, welche nach dem vorliegenden Verfahren epoxidiert werden können, beispielsweise die folgenden in Betracht:

Ethylen, Propylen, die Butene, Butadien, die Pentene, Isopren, Hexen(1), Hexen(3), Hepten(1), Okten(1), Diisobutylen, Nonen(1), Tetradecen(1), Pentamyrcen, Camphen, Undecen(1), Dodecen(1), Tridecen(1), Tetradecen(1), Pentadecen(1), Hexadecen(1), Heptadecen(1), Oktadecen(1), Nonadecen(1), Eikosen(1), die Trimeren und Tetrameren des Propylens, die Polybutadiene, die Polyisoprene, Styrol, α-Methylstyrol, Divinylbenzol, Inden, Stilben, Cyclopenten, Cyclohexen, Cyclohepten, Cyclooocten, Cyclooctadien, Cyclododecen, Cyclododekatrien, Dicyclopentadien, Methylencyclopropan, Methylencyclopentan, Methylencyclohexan, Vinylcyclohexen, Methallylketon, Allylchlorid, Allylbromid, Arylsäure, Methacrylsäure, Crotonsäure, Vinylessigsäure, Crotylchlorid, Methallylchlorid, die Dichlorbutene, Allylalkohl, Allylkarbonat, Allylacetat, die Alkyle der Acrylate und Methacrylate, Diallylmaleat, Diallylphthalat, die ungesättigten Öle wie Sojaöl, die ungesättigten Fettsäuren wie Ölsäure, Linolensäure, Balidinsäure, Erucasäure, Oleostearinsäure, Myristinsäure, Palmitinölsäure, Ricinolsäure und deren Ester.

Ein Vorteil der Erfindung besteht darin, daß sich hierbei das als Reaktand benötigte Wasserstoffperoxid in allen handelsüblichen Formen verwenden läßt, nämlich in Form wäßriger Wasserstoffperoxidlösungen mit einem Wasserstoffperoxidgehalt von 30 bis 90.Gew.-% oder als reines Wasserstoffperoxid, stärker verdünntes Wasserstoffperoxid, in organischen Lösungsmitteln gelöstes wasserfreies Wasserstoffperoxid oder in Form von Verbindungen, die unter den Reaktionsbedingungen Wasserstoffperoxid freisetzen (Metallperoxide, wie Magnesium- oder Zinkperoxid sowie Wasserstoffperoxid-Anlagerungsverbindungen (Peroxohydrate), z.B. von Natriumcarbonat, Natriumpyrophosphat und Harnstoff).

Besonders vorteilhaft ist, wenn man als Reaktionsmedium ein organisches Lösungsmittel oder ein Lösungsmittelgemisch verwendet, welches einen Übertritt von als wäßrige Lösung eingesetztem Wasserstoffperoxid in die organische Phase gestattet.

Als organische Lösungsmittel können dafür z.B. Alkyl- oder Cycloalkylester von gesättigten aliphatischen Carbonsäuren mit einer Kohlenstoffzahl von 4–8 (siehe z.B. DE-PS 3 225 307, Seite 5), Methylenchlorid, Dioxan, tert.-Butanol, Tetrahydrofuran, Benzol, Äthanol, Chloroform und Methanol verwendet werden.

Als Lösungsmittelgemische kommen z.B. Kombinationen aus einem oder mehreren der obengenannten Carbonsäureester mit Wasser, Methylenchlorid, Dioxan, tert.-Butanol, Tetrahydrofuran, Benzol, Äthanol, Chloroform und/oder Methanol in Frage.

Für die Lösung von wasserfreiem Wasserstoffperoxid hat sich ein Einsatz von Alkyl- oder Cycloalkylestern von gesättigten aliphatischen Carbonsäuren mit einer Kohlenstoffzahl von 4–8 besonders bewährt.

Die anzuwendenden Mengen an Katalysator, die im erfindungsgemäßen Verfahren eingesetzt werden, können in einem weiten Bereich liegen. Die im Einzelfall anzuwendende Katalysatorkonzentration kann, entsprechend dem Typ der gewählten, gemäß Erfindung vorgesehenen Wolframporphyrinverbindung sowie entsprechend der Reaktivität des jeweils umzusetzenden Olefins gewählt werden.

Sie liegt in einem Konzentrationsbereich, der im allgemeinen 1/10000 bis 1/2 mol, vorzugsweise 1/5000 bis 1/5 mol pro mol Wasserstoffperoxid beträgt.

Ein weiterer Gegenstand der Erfindung ist die mehrfache Verwendung des gebrauchten Katalysators, der nach geeigneter Abtrennung des Reaktionsgemisches für weitere Ansätze eingesetzt werden kann.

Nach einer anderen vorteilhaften Ausführungsform der Erfindung können die ohnehin hohen Selektivitätsraten durch Zusatz von geringen Mengen, vorzugsweise von 0,1 bis 10 mol eines heterocyclischen Amins, bezogen auf 1 mol des Katalysators, insbesondere von äquimolaren Mengen, einer Verbindung aus der Familie des Pyridins, wie 2,6-Dimethylpyridin, 2,6-Ditertiärbutylpyridin, 3,5-Dimethylpyridin sowie die drei Picoline, die 4-Halogenopyridine sowie die Salze des 2,2-Bipyridyls oder des Imidazols, z.B. des Imidazol selbst, verbessert werden. Die Konzentration des Olefins im Reaktionssystem ist nicht kritisch, das molare Verhältnis zwischen Olefin und Wasserstoffperoxid kann 1:30 bis 30:1 betragen.

Die Reaktionstemperaturen können in einem breiten Bereich liegen. Sie hängen ab von der jeweiligen Aktivität des verwendeten Katalysators, der Reaktivität des verwendeten Olefins, der Neigung des gewünschten Oxirans zur Ringöffnung und der Art des Lösungsmittels. Sie liegen im allgemeinen bei 0 bis 150°, vorzugsweise 20 bis 120°, insbesondere 20 bis 80°C. Die Reaktionszeiten liegen normalerweise bei 10 Minuten bis 24 Stunden. Die Reaktionen können unter Atmosphärendruck oder bei höheren Drucken durchgeführt werden, solange das Reaktionssystem in flüssiger Phase gehalten werden kann.

Vorzugsweise wird in einem Druckbereich zwischen 1 und 50 bar gearbeitet.

Die mit der Erfindung erzielbaren Vorteile sind:

- Sehr kurze Reaktionszeiten
- Hohe Selektivität (kaum Nebenprodukt)
- Niedrige Katalysatorkonzentration
- Hohe chemische Stabilität des Katalysators, insbesondere gegenüber dem Epoxidationsmittel
- Keine oder nur minimale $H_2O_2$-Zersetzung

– Aus dem Epoxidationsmittel entsteht nur Wasser
– Leichte Abtrennbarkeit und Wiederverwendbarkeit des Katalysators
– Einfacher Verfahrensweg

Die Erfindung wird im folgenden anhand von Ausführungsbeispielen näher erläutert.

Vorbemerkung zu den einzelnen Beispielen:

Die in den Ausführungsbeispielen verwendeten, zum Teil nach der Parallelanmeldung oder nach der Literatur erhältlichen Katalysatoren werden zur Epoxidation unterschiedlicher olefinischer Ausgangsstoffe mit Wasserstoffperoxid gemäß der Erfindung wie folgt eingesetzt:

Man stellt eine Lösung aus Olefin, Katalysator und Lösungsmittel her, erwärmt sie auf eine Temperatur im Bereich von 20–100°C und versetzt mit Wasserstoffperoxid (30 bis 90 Gew.-%) – Version I – oder man stellt eine Lösung aus Olefin, Wasserstoffperoxid (30 abis 90 Gew.-%) und Lösungsmittel her und versetzt diese dann mit Katalysator; dann wird unter Rühren und Erwärmen umgesetzt – Version II.

In einer abgewandelten Ausführungsform, die sich auf die Version I und II gleichermaßen anwenden läßt, wird den vorgelegten Komponenten vor Zugabe der restlichen Komponente noch eine kleine Menge eines heterocyclischen Amins, z.B. aus der Familie des Pyridins oder Imidazols zugesetzt. Bei den eingesetzten Olefinen kann unter atmosphärischem Druck gearbeitet werden. Im Verlaufe der Umsetzung werden Proben entnommen und auf ihren Gehalt an Epoxid bzw. $H_2O_2$ analysiert. Die Menge an gebildeten Epoxiden wird entweder durch Gaschromatographie oder Titration, diejenige an Wasserstoffperoxid durch übliche Titration mit Cer(IV)-sulfat ermittelt. Die bei den Versuchen erhaltenen Ergebnisse gehen aus der folgenden Tabelle hervor, wobei die Selektivität wie folgt definiert ist:

$$\text{Selektivität (\%)} = \frac{\text{Mol gebildetes Epoxid}}{\text{Mol umgesetztes } H_2O_2} \times 100$$

| Beispiel Nr. | Olefin (mol) | $H_2O_2$ (mol) | Katalysator | (mol) | Reaktionsbeding. Solvent (ml) | Temp. (°C) | Zeit (h) | Umsatz $H_2O_2$ (%) | Selektiv. % |
|---|---|---|---|---|---|---|---|---|---|
| 1 | 1,5-COD[1] (72) | 25,86% in nPAC[2] (24) | OW(TTP)Cl[3] | $24 \times 10^{-2}$ | nPAC | 60 | 1 | 92,5 | 83,2 |
| 2 | 1,5-COD (72) | 25,86% in nPAC (24) | OW(TTP)Cl* | $24 \times 10^{-2}$ | nPAC | 60 | 0,5 | 94,2 | 96,6 |
| 3** | 1,5-COD (72) | - | OW(TTP)Cl | $24 \times 10^{-2}$ | nPAC | 60 | 1 | 0 | 0 |
| 4 | 1,5-COD (72) | 85 % in $H_2O$ (24)[2] | OW(TPP)Br[4] | $24 \times 10^{-2}$ | nPAC | 60 | 1 | 97,2 | 98,9 |
| 5 | 2-Methyl-2-buten (72) | 25,86% in nPAC (24) | OW(TPP)Br | $24 \times 10^{-2}$ | nPAC | 60 | 3 | 94,6 | 70,4 |
| 6 | 1,5-COD | 25,86% in nPAC (24) | OW(TPP)Br | $24 \times 10^{-2}$ | nPAC | 60 | 1 | 96,1 | 86,5 |
| 7 | Cyclohexen | 85 % in $H_2O$ (24)[2] | OW(TPP)Br | $24 \times 10^{-2}$ | tert.-Butanol | 60 | 1,5 | 83,3 | 81,1 |

1) 1,5-Cyclooctadien
2) Essigsäurepropylester
3) TTP = 5,10,15,20-Tetra(p-tolyl)-porphyrin
4) TPP = 5,10,15,20-Tetraphenyl-porphyrin

* Wiederverwendung des Katalysators nach Beispiel 1
** Kontrollbeispiel

Die folgenden weiteren Ausführungsbeispiele zur Epoxidation verschiedener Olefine verwenden Katalysatorstoffe, deren Herstellung vor der Ergebnistabelle zusammengestellt ist.

Beispiel 8

Herstellung von Perchlorato-oxo[5,10,15,20-tetra(p-chlorphenyl)porphyrinato]wolfram(V), WO(Tp-ClPP)$OClO_3$

Eine Lösung von 232 mg (0.12 mmol) $[WO(Tp\text{-}ClPP)]_2O$ in 15 ml $CHCl_3$ wurde mit 2 ml 7% wäßriger $HClO_4$ versetzt. Unter ständigem Rühren erwärmte man leicht, bis das $CHCl_3$ verdunstet war. Der ausgefallene Komplex wurde abfiltriert, mit destilliertem Wasser neutral gewaschen und aus $CHCl_3$ kristallisiert. Man erhielt 225 mg (88%) WO(Tp-ClPP)$OClO_3$ in Form von schwarzen Kristallen.

$C_{44}H_{24}N_4Cl_5O_5W$ (1049.8)

Ber.: C 50.35 H 2.28 N 5.33; C:H = 22.08; C:N = 9.44

Gef.: C 45.77 H 1.99 N 4.74; C:H = 23.0; C:N = 9.65

Substanz enthielt noch Aluminiumoxid

UV/VIS (λ max, log ε): 314(4.50), 456(5.26), 586(4.07), 628(3.78) nm

IR (KBr): ca. 1100 $cm^{-1}$ (Perchlorat- und W=O Banden liegen unter Porphyrinbanden)

Beispiel 9/1

Herstellung von Perchloratooxo(2,3,7,8,12,13,17,18-octaethylporphyrinato)wolfram(V), WO(OEP)$OClO_3$

Eine Lösung von 222 mg (0.15 mmol) $[WO(OEP)]_2O$ in 30 ml $CHCl_3$ wurde mit 3 ml Perchlorsäure (7% wäßrige Lösung) über Nacht gerührt. Das Lösungsmittel wurde unter gelindem Erwärmen entfernt und der Rückstand mit destilliertem Wasser neutral gewaschen. Kristallisation aus Toluol lieferte 215 mg (86%) WO(OEP)$OClO_3$ als violette, metallisch glänzende Plättchen.

$C_{36}H_{44}N_4O_5WCl$ (832.1)

Ber.: C 51.97 H 5.33 N 6.73

Gef.: C 51.32 H 5.47 N 7.07

UV/VIS (λ max, log ε): 328(4.52), 438(4.94), 562(4.00) 598(3.83) nm

IR (KBr): ca. 1100 $cm^{-1}$ (W=O Bande liegt unter Porphyrinbanden)

MS (FD): 831 ($^{184}WO(OEP)^+OClO_3^+$,

Beispiel 10/1

Herstellung von μ-Oxobis[oxo{5,10,15,20-tetra(p-methoxyphenyl)porphyrinato} wolfram(V)], $[WO(TAP)]_2O$

Eine Lösung von 258 mg (0.5 mmol) $WBr_5$ in 30 ml Benzonitril wurde 1.5 h unter Rückfluß erhitzt und nach Zugabe von 170 mg (0.25 mmol) $H_2(TAP)$ weitere 3.5 h am Sieden gehalten. Nach Abdestillieren des Lösungsmittels im Hochvakuum wurde der Rückstand in $CHCl_3$ aufgenommen und an $Al_2O_3$ (Aktivität II, basisch, 2.6 x 4.5 cm) chromatographiert.

1. Fraktion: rotviolett $H_2(TAP)$ (Eluens: $CH_2Cl_2$)
2. Fraktion: grün WO(TAP)X (Eluens: $CH_2Cl_2$/MeOH 99/1)
3. Fraktion: grün WO(TAP)X (Eluens: $CH_2Cl_2$/MeOH 95/5)

Da die Fraktionen 2 und 3 im UV/VIS-Spektrum dieselben Banden zeigten, wurden sie vereinigt und das Lösungsmittel im Vakuum abdestilliert. Der Rückstand wurde in $CHCl_3$ gelöst, mit 20 ml verd. wäßriger KOH versetzt und 2 h gerührt. Das $CHCl_3$ wurde durch gelindes Erwärmen entfernt, der ausgefallene Niederschlag abfiltriert und mit destilliertem Wasser neutral gewaschen. Man erhielt 70 mg (15%) grüne Schüppchen von $[WO(TAP)]_2O$.

UV/VIS (λ max, log ε): 318(4.76), 450(5.54), 470(5.07), 588(4.15), 632(4.20), 672(3.99) nm

IR (KBr): 650, 719 $cm^{-1}$ (typisch für μ-oxo-Systeme)

Beispiel 11/1

Herstellung von μ-Oxobis[oxo(2,3,7,8,12,13,17,18-octaethylporphyrinato)wolfram(V)], $[WO(OEP)]_2O$

In einem 100 ml-Zweihalskolben mit Rückflußkühler und Stickstoffeinleitungsrohr wurden 641 mg (1.2 mmol) $H_2(OEP)$, 1.3 g (2.2 mmol) $WBr_5$ in 75 ml 1.2.4-Trichlorbenzol unter Rühren zum Sieden erhitzt. Fortlaufend entnommene Proben wurden UV/VIS-spektroskopisch untersucht. Die Spektren nach 3 h bzw. 4.5 h Reaktionszeit unterschieden sich nicht und ließen nur noch geringe Spuren freien Porphyrins erkennen. Die Reaktion wurde abgebrochen und das Lösungsmittel im Hochvakuum entfernt. Der dunkelbraune Rückstand wurde in $CH_2Cl_2$ aufgenommen und filtriert. Man chromatographierte an $Al_2O_3$ (Aktivität III-n, 3.8 x 13 cm):

1. Fraktion, grünlich-hellblau, nach Verlassen der Säule allmählich nach rot umschlagend, $H_2(OEP)$, 10 mg, Eluens $CH_2Cl_2$;

2. Fraktion, grün, Eluens $CH_2Cl_2$/MeOH 95:5, MeOH-Anteil allmählich auf 8% gesteigert. Die 2. Fraktion wurde zur Trocknen eingedampft, in 40 ml $CH_2Cl_2$ aufgenommen und mit einer Lösung von 2 g KOH in 20 ml Wasser 18 h gerührt. Man entfernte das Lösungsmittel durch gelindes Erwärmen, filtrierte den ausgefallenen Komplex ab und wusch mit destilliertem Wasser neutral. Kristallisation aus $CH_2Cl_2$/Toluol lieferte 764 mg (86%) μ-oxo-Komplex $[WO(OEP)]_2O$ als rot-violettes feinkristallines Pulver.

$C_{72}H_{88}N_8O_3W_2$ (1481.2)
UV/VIS (λ max, log ε): 366(4.74), 430(5.09), 558(4.11), 660(3.32) nm
IR (KBr): 646, 725 $cm^{-1}$ (typisch für μ -oxo-Systeme)

Beispiel 12

Herstellung von Acetatooxo(2,3,7,8,12,13,17,18-octaethylporphyrinato)wolfram(V), WO(OEP)OAc

Eine Lösung von 222 mg (0.15 mmol) $[WO(OEP)]_2O$ in 30 ml $CHCl_3$ wurde mit 2 ml Eisessig und 50 mg Natriumacetat über Nacht gerührt. Dann entfernte man das Lösungsmittel und Teile der Essigsäure durch leichtes Erwärmen. Waschen mit destilliertem Wasser befreite den Komplex von anhaftender Essigsäure und Natriumacetat. Nach Trocknung im Vakuum und Kristallisation aus Toluol resultierten 202 mg (85%) WO(OEP)OAc als violett schimmernde, plättchenförmige Kristalle.

$C_{38}H_{47}N_4O_3W$ (791.67)
Ber.: C 57.65 H 5.98 N 7.08
Gef.: C 56.95 H 5.63 N 7.15
UV/VIS (λ max, log ε): 324(4.45), 442(5.01), 565(4.00), 600(3.77) nm
IR (KBr): 1660 (asymm. COO), 935 (W = O) $cm^{-1}$
MS (FD): 791 ($^{184}WO(OEP)OAc^+$)

Beispiel 13

Herstellung von Chlorooxo(5,10,15,20-tetraphenylporphyrinato)wolfram(V), WO(TPP)Cl

Eine Lösung von 246 mg (0.15 mmol) $[WO(TPP)]_2O$ in 30 ml $CHCl_3$ wurde mit 4 ml 37% wäßriger Salzsäure über Nacht gerührt. Nach Verdampfen des Lösungsmittels wurde der Rückstand mit destilliertem Wasser neutral gewaschen. Kristallisation aus Toluol lieferte 229 mg (90%).

WO(TPP)Cl als dunkelgrün metallisch schimmernde Kristalle.

$C_{44}H_{28}N_4OWCl$ (832.1)
Ber.: C 62.32 H 3.33 N 6.61
Gef.: C 64.5 H 3.41 N 6.84
UV/VIS (λ max, log ε): 316(4.57), 466(5.23), 600(3.95), 632(3.90) nm
IR (KBr): 950 $cm^{-1}$ (W = O)
MS (FD): 847 ($^{184}WO(TPP)Cl^+$), 812 ($^{184}WO(TPP)^+$)

Beispiel 14

Herstellung von Methoxo-oxo[5,10,15,20-tetra(p-chlorophenyl)-porphyrinato]wolfram(V), WO(TP-ClPP)OMe

316 mg (0.164 mmol) $[WO(Tp-ClPP)]_2O$ wurden in 30 ml $CHCl_3$ gelöst und mit 20 ml MeOH zum Sieden erhitzt. Danach wurde das Lösungsmittel im Vakkum entfernt und der Rückstand aus MeOH umkristallisiert. Man erhielt 240 mg (75%) grüne Plättchen des Methoxokomplexes.

$C_{45}H_{27}N_4Cl_4O_2W$ (981.4)
Ber.: C 55.08 H 2.75 N 5.71
Gef.: C 52.46 H 2.36 N 5.46
(Substanz enthielt noch Aluminiumoxid, da aus μ-oxo-Komplex dargestellt)
UV/VIS (λ max, log ε): 324(4.52), 444(4.79), 466(5.35), 578(4.04), 620(3.83) nm
IR (KBr): 2795 (OMe), 920 (W = O) $cm^{-1}$
MS (FD): 979 ($^{184}WO(Tp-ClPP)OMe^+$)

Beispiel 15

Herstellung von Acetatooxo[5,10,15,20-tetra(p-tolyl)-porphyrinato]wolfram(V), WO(TTP)OAc

Eine Lösung von 263 mg (0.15 mmol) $[WO(TTP)]_2O$ in 30 ml $CHCl_3$ wurde mit 2 ml Eisessig sowie 50 mg Natriumacetat versetzt und über Nacht gerührt. Man entfernte das Lösungsmittel und Teile der Essigsäure durch gelindes Erwärmen. Der Rückstand wurde durch Waschen mit destilliertem Wasser vom Natriumacetat und anhaftender Essigsäure befreit, im Vakuum getrocknet und aus Toluol kristallisiert. Man erhielt 248 mg (99%) WO(TTP)OAc als feinkristallines dunkelgrünes Kristallpulver.

$C_{50}H_{39}N_4O_3W$
Ber.: C 64.73 H 4.24 N 6.04
Gef.: C 67.17 H 4.29 N 6.48

UV/VIS (λ max, log ε): 306(4.49), 456(5.27), 590(3.96), 632(3.90) nm
IR (KBr): 1660 (asymm. COO), 950 (W = O) $cm^{-1}$
MS (FD): 868 ($^{184}WO(TTP)^{+}$)

Beispiel 16

Herstellung von Chlorooxo[5,10,15,20-tetra(p-tolyl)-porphyrinato]wolfram(V), WO(TTP) Cl
Eine Lösung von 263 mg (0.15 mmol) $[WO(TTP)]_2O$ in 30 ml $CHCl_3$ wurde mit 4 ml 37% wäßriger Salzsäure über Nacht gerührt. Nach Abdampfen des Lösungsmittels wurde der Rückstand mit destilliertem Wasser neutral gewaschen. Kristallisation aus Toluol ergab 255 mg (94%) WO(TTP)Cl als dunkelgrüne schuppige Kristalle.
$C_{48}H_{36}N_4OWCl$ (904.14)
Ber.: C 63.74% H 4.01% N 6.20%
Gef.: C 66.79% H 3.99% N 6.56%
UV/VIS (λ max, log ε): 314(4.60), 470(5.26), 604(3.95), 650(4.00) nm
IR: 950 $cm^{-1}$, (W = O)
MS: 904, ($^{148}WO(TTP)Cl$)

Beispiel 17

Herstellung von Perchloratooxo[5,10,15,20-tetra(p-tolyl)-porphyrinato]wolfram(V), $WO(TTP)OClO_3$
Eine Lösung von 264 mg (0.15 mmol) $[WO(TTP)]_2O$ in 30 ml $CHCl_3$ wurde mit 4 ml wäßriger 7% Perchlorsäure 18 h gerührt. Man dampfte das Lösungsmittel unter leichtem Erwärmen ab und wusch den Rückstand mit destilliertem Wasser neutral. Kristallisation aus Toluol lieferte 247 mg (85%) $WO(TTP)ClO_4$ als tief-violette metallisch glänzende Plättchen.
$C_{48}H_{36}N_4O_5WCl$ (968.1)
Ber.: C 59.55 H 3.75 N 5.79
Gef.: C 61.78 H 3.84 N 6.40
UV/VIS (λ max, log ε): 308(4.49), 458(4.92), 632(3.72), 650(4.00) nm
IR (KBr): ca. 1100 $cm^{-1}$ (Bande für koordiniertes Perchlorat liegt unter Porphyrinbanden)
MS (FD): 868 ($^{184}WO(TTP)^{+}$)

Ergebnistabelle

| Beispiel Nr. | Olefin (mol) | $H_2O_2$ (mol) | Katalysator | (mol) | Reaktionsbeding. Solvent (ml) | Temp. (°C) | Zeit (h) | Umsatz $H_2O_2$ (%) | Selektiv. % |
|---|---|---|---|---|---|---|---|---|---|
| 8 | 1,5-COD (36) | 85% aq. (12) | OW(Tp-ClPP)OClO$_3$ | $12 \times 10^{-2}$ | n-PAC | 60 | 1 | 87,5 | 53,4 |
| 9/1 | 1,5-COD (36) | 85% aq. (12) | OW(OEP)OClO$_3$ | $12 \times 10^{-2}$ | n-PAC | 60 | 1 | 96,7 | 72,1 |
| 9/2 | 1,5-COD (36) | 85% aq. (12) | aus Nr. 9/1 (rezykliert) | $12 \times 10^{-2}$ | n-PAC | 60 | 1 | 92,4 | 86,1 |
| 10/1 | 1,5-COD (18) | 85% aq. (6) | [OW(TAP)]$_2$O | $6 \times 10^{-2}$ | n-PAC | 60 | 1 | 90,5 | 62,1 |
| 10/2 | 1,5-COD (18) | 85% aq. (6) | aus Nr. 10/1 (rezykliert) | $6 \times 10^{-2}$ | n-PAC | 60 | 1 | 88,7 | 70,9 |
| 11/1 | 1,5-COD (36) | 85% aq. (12) | [OW(OEP)]$_2$O | $12 \times 10^{-2}$ | n-PAC | 60 | 1 | 97,9 | 33,7 |
| 11/2 | 1,5-COD (36) | 85% aq. (12) | aus Nr. 11/1 (rezykliert) | $12 \times 10^{-2}$ | n-PAC | 60 | 3 | 97,3 | 94,2 |
| 12 | 1,5-COD (36) | 85% aq. (12) | OW(OEP)OAc | $12 \times 10^{-2}$ | n-PAC | 60 | 3 | 99,4 | 69,1 |
| 13 | 1,5-COD (36) | 85% aq. (12) | OW(TPP)Cl | $12 \times 10^{-2}$ | n-PAC | 60 | 4 | 25,9 | 64,4 |
| 14 | 1,5-COD (36) | 85% aq. (12) | OW(Tp-ClPP)OMe | $12 \times 10^{-2}$ | n-PAC | 60 | 1 | 94,5 | 66,7 |

Ergebnistabelle

| Beispiel Nr. | Olefin (mol) | $H_2O_2$ (mol) | Katalysator | (mol) | Reaktionsbeding. Solvent (ml) | Temp. (°C) | Zeit (h) | Umsatz $H_2O_2$ (%) | Selektiv. % |
|---|---|---|---|---|---|---|---|---|---|
| 15 | 1,5-COD (36) | 85% aq. (12) | OW(TTP)OAc | $12 \times 10^{-2}$ | n-PAC | 60 | 1 | 93,6 | 71,2 |
| 16 | 1,5-COD (36) | 85% aq. (12) | OW(TTP)Cl | $12 \times 10^{-2}$ | n-PAC | 60 | 4 | 95,6 | 81,2 |
| 17 | 1,5-COD (36) | 85% aq. (12) | $OW(TTP)OClO_3$ | $12 \times 10^{-2}$ | nPAC | 60 | 1 | 97,0 | 49,2 |
| 18 | 1,5-COD | 30% aq. | OW(TPP)Br | $12 \times 10^{-2}$ | t-Butanol | 60 | 4 | 94,7 | 60,2 |

## Patentansprüche

1. Verfahren zur katalytischen Epoxidation von Olefinen mit Wasserstoffperoxid, in Gegenwart von Metallporphyrinkomplexen, dadurch gekennzeichnet, daß man das Olefin in Gegenwart von
– Wolfram-oxo-Komplexen oder
– einer zweikernigen Verbindung des Typs μ-Oxobis[porphyrinatooxowolfram(V)] oder
– einer Oxoperoxoverbindung des Typs cis-Oxoperoxowolfram(VI)porphyrin oder
– eines cis-Dioxoporphyrinatowolfram(VI)-Komplexes
mit jeweils
– Oktaethylporphyrin oder
– 5,10,15,20-Tetraphenylporphyrin oder
5,10,15,20-Tetra(4-pyridyl)-porphyrin
als Liganden, bei denen gegebenenfalls jeweils Wasserstoffatome oder freie Elektronenpaare an den Phenyl- bzw. Pyridylgruppen ein- oder mehrmals substituiert sind durch Halogen, Hydroxy, Carboxy, Cyano, Rhodano, Nitro, $C_1$–$C_6$-Alkyl, Trihalogenmethyl, $C_1$–$C_6$-Alkoxy, $C_1$–$C_6$-Alkansulfonyloxy, Aminocarbonyl, einen oder zwei $C_1$–$C_6$-Alkylreste enthaltendes Aminocarbonyl, $C_1$–$C_6$-Alkylcarbonyl, Amino, Di-$C_1$-$C_6$-Alkylamino, $C_1$–$C_6$-Alkanoylamino, $C_1$–$C_6$-Alkyl-$C_1$–$C_6$-alkanoylamino, $C_1$–$C_6$-Alkansulfonylamino, $C_1$–$C_6$-Alkyl-$C_1$–$C_6$-alkansulfonylamino, Aminosulfonyl, einen oder zwei $C_1$–$C_6$-Alkylreste enthaltendes Aminosulfonyl, $C_1$–$C_6$-Alkoxysulfonyl(–$SO_2$–O–$C_1$–$C_6$–Alkyl), Sulfo oder $C_1$–$C_6$-Alkansulfonyl und zwei dieser Reste auch die Methylendioxygruppe sein können, wobei der Komplex bei den Wolfram-oxo-Komplexen gegebenenfalls am Zentralatom ein Anion aus der Reihe $F^-$, $Cl^-$, $Br^-$, $J^-$, $CH_3O^-$, $C_2H_5O^-$, $C_3H_7O^-$, t-$C_4H_9O^-$, $HO^-$, $AcO^-$, $SCN^-$, $CN^-$, $C_6H_5O^-$, trägt, in homogener Phase oder in einem Zweiphasensystem mit Wasserstoffperoxid umsetzt.

2. Verfahren nach Anspruch 1, daduch gekennzeichnet, daß man als Reaktionsmedium ein organisches Lösungsmittel oder ein Lösungsmittelgemisch verwendet, welches einen Übertritt von als wäßrige Lösung eingesetztem Wasserstoffperoxid in die organische Phase gestattet.

3. Verfahren nach Anspruch 1 oder 2, dadurch gekennzeichnet, daß man bei Einsatz von wasserfreiem Wasserstoffperoxid als organische Lösungsmittel Alkyl- oder Cycloalkylester von gesättigten aliphatischen Carbonsäuren mit einer Kohlenstoffzahl von 4–8 einsetzt.

4. Verfahren nach den Ansprüchen 1 bis 3, dadurch gekennzeichnet, daß die Umsetzung in Gegenwart von 0,1 bis 10 mol, vorzugsweise 0,5–5 mol, bezogen auf 1 mol des Katalysators, einer Verbindung aus der Familie des Pyridins oder Imidazols, erfolgt.

5. Verfahren nach Anspruch 4, dadurch gekennzeichnet, daß γ-Picolin oder Imidazol eingesetzt wird.

6. Verfahren nach den Ansprüchen 1 bis 5, daduch gekennzeichnet, daß man den zur Epoxidation verwendeten, gebrauchten Katalysator nach Abtrennung der Reaktionsmischung für weitere Epoxidationen einsetzt.

## Claims

1. A process for the catalytic epoxidation of olefins with hydrogen peroxide in the presence of metal porphyrin complexes, characterized in that the olefin is reacted with hydrogen peroxide in homogeneous phase or in a two-phase system in the presence of
– tungsten-oxo complexes or
– a binuclear compound of the μ-oxo-bis-[porphyrinato-oxo-tungsten(V)] type or
– an oxo-peroxo compound of the cis-oxo-peroxotungsten(VI) porphyrin type or
– a cis-dioxoporphyrinatotungsten(VI) complex with – in each case–
– octaethyl porphyrin or
– 5,10,15,20-tetraphenylporphyrin or
– 5,10,15,20-tetra(4-pyridyl) porphyrin
as ligands in which hydrogen atoms or free electron pairs at the phenyl or pyridyl groups are optionally substituted one or more times by halogen, hydroxy, carboxy, cyano, thiocyano, nitro, $C_1$–$C_6$ alkyl, trihalomethyl, $C_1$–$C_6$ alkoxy, $C_1$–$C_6$ alkanesulfonyloxy, aminocarbonyl, aminocarbonylcontaining one or two $C_1$–$C_6$ alkyl groups, $C_1$–$C_6$ alkyl carbonyl, amino, di-$C_1$–$C_6$-alkylamino, $C_1$–$C_6$ alkanoylamino, $C_1$–$C_6$-alkyl-$C_1$–$C_6$-alkanoylamino, $C_1$–$C_6$-alkanesulfonylamino, $C_1$–$C_6$-alkyl-$C_1$–$C_6$-alkanesulfonylamino, aminosulfonyl, aminosulfonyl containing one or two $C_1$–$C_6$ alkyl groups, $C_1$–$C_6$ alkoxysulfonyl (–$SO_2$–O–$C_1$–$C_6$–alkyl), sulfo or $C_1$–$C_6$ alkanesulfonyl and two of these radicals may also be the methylenedioxy group, the complex optionally containing an anion from the series $F^-$, $Cl^-$, $Br^-$, $I^-$, $CH_3O^-$, $C_2H_5O^-$, $C_3H_7O^-$, t-$C_4H_9O^-$, $HO^-$, $AcO^-$, $SCN^-$, $CN^-$, $C_6H_5O^-$ at the central atom in the case of the tungsten-oxo complexes.

2. A process as claimed in claim 1, characterized in that the reaction medium used is an organic solvent or solvent mixture which enables hydrogen peroxide used in the form of an aqueous solution to pass into the organic phase.

3. A process as claimed in claim 1 or 2, characterized in that, where anhydrous hydrogen peroxide is

used, alkyl or cycloalkyl esters of saturated aliphatic carboxylic acids containing 4 to 8 carbon atoms are used as the organic solvent.

4. A process as claimed in claims 1 to 3, characterized in that the reaction is carried out in the presence of 0.1 to 10 mol and preferably 0.5 to 5 mol, based on 1 mol of the catalyst, of a compound from the pyridine or imidazole family.

5. A process as claimed in claim 4, characterized in that γ-picoline or imidazole is used.

6. A process as claimed in claims 1 to 5, characterized in that the spent catalyst used for the epoxidation is used for further epoxidations after separation of the reaction mixture.

## Revendications

1. Procédé d'époxydation catalytique d'oléfines à l'aide de peroxyde d'hydrogène en présence de complexes métalliques de porphyrine, caractérisé en ce que l'on fait réagir l'oléfine en présence de:
- complexes oxo-tungsténiques ou (
- d'un composé bicyclique du type μ-oxo-bis-[porphyrinato-oxo-tungstène(V)] ou
- d'un composé oxoperoxo du type cis oxoperoxotungstène(VI)porphyrine ou
- d'un complexe cis dioxoporphyrinatotungstène(VI) avec respectivement
- de l'octaéthylporphyrine ou
- de la 5,10,15,20-tétraphénylporphyrine ou
- de la 5,10,15,20-tétra(pyridyl-4)porphyrine

en tant que ligands pour lesquels éventuellement chacun des hydrogènes ou des paires d' électrons libres sur les radicaux phényle ou pyridyle, sont substitués une ou plusieurs fois par un halogène, un hydroxy, un carboxy, un cyano, un isocyanato, un nitro, un alcoyle en $C_1$–$C_6$, un trihalogénométhyl, un alcoxy en $C_1$–$C_6$, un alcane en $C_1$–$C_6$ sulfonyloxy, un aminocarbonyl, un aminocarbonyl contenant un ou deux restes alcoyle en $C_1$–$C_6$, un alcoyl en $C_1$–$C_6$ carbonyl, un amino, un dialcoyl en $C_1$–$C_6$ amino, un alcanoyl en $C_1$–$C_6$-amino, un (alcoyl en $C_1$–$C_6$) (alcanoyl en $C_1$–$C_6$)-amino, un alcane en $C_1$–$C_6$ sulfonylamino, un (alcoyl en $C_1$–$C_6$) (alcane en $C_1$–$C_6$) sulfonylamino, un aminosulfonyl, un aminosulfonyl contenant un ou deux radicaux alcoyle en $C_1$–$C_6$, un (alcoxy en $C_1$–$C_6$) sulfonyl [$SO_2$–O($C_1$–$C_6$)alcoyl], un sulfo, ou un (alcane $C_1$–$C_6$) sulfonyl et deux de ces radicaux peuvent être également le groupe méthylènedioxy dans lesquels le complexe pour les complexes oxo-tungstène porte le cas échéant un anion choisi dans le groupe de $F^-$, $Cl^-$, $Br^-$, $I^-$, $CH_3O^-$, $C_2H_5O^-$, $C_3H_7O^-$, t-$C_4H_9O^-$, $HO^-$, $AcO^-$, $SCN^-$, $CN^-$, $C_6H_5O^-$ en phase homogène ou dans un système à deux phases avec le peroxyde d'hydrogène.

2. Procédé selon la revendication 1, caractérisé en ce que l'on utilise comme milieu réactionnel un solvant ou un mélange de solvants organiques qui permettent le passage du peroxyde d'hydrogène mis en oeuvre sous forme de solution aqueuse, dans la phase organique.

3. Procédé selon la revendication 1 ou 2, caractérisé en ce que l'on met en Ôuvre lors de l'utilisation de peroxyde d'hydrogène anhydre, en tant qe solvant organique un ester d'alcoyle ou de cycloalcoyle d'acides carboxyliques aliphatiques saturés ayant un nombre de carbone allant de 4 à 8.

4. Procédé selon les revendications 1 à 3, caractérisé en ce que la réaction s'effectue en présence de 0, 1 à 10 mol, de préférence 0, 5 à 5 mol, rapporté à 1 mole de catalyseur, d'un composé choisi dans la famille de la pyridine ou de l'imidazole.

5. Procédé selon la revendication 4, caractérisé en ce que l'on met en Ôuvre de la γ-picoline ou de l'imidazole.

6. Procédé selon les revendications 1 à 5, caractérisé en ce que l'on met en Ôuvre le catalyseur épuisé, utilisé pour l'époxydation après séparation du mélange réactionnel, pour de nouvelles époxydations.